# EUROPÄISCHE PATENTANMELDUNG

(11) **EP 1 234 542 A1**
(43) Veröffentlichungstag der Anmeldung: **28.08.2002**
(21) Anmeldenummer: 02002897.3
(22) Anmeldetag: 08.02.2002
(51) Int. Cl.: A61B 6/04, A61B 6/12

(54) **Vorrichtung und Verfahren zur Bestimmung der räumlichen Beziehung von einander unabhängig aufgenommenen Röntgendatensätzen**

(30) Priorität: 22.02.2001 DE 10108633
(71) Anmelder: SIEMENS AKTIENGESELLSCHAFT, 80333 München (DE)
(72) Erfinder: Graumann, Rainer, Dr., 91315 Höchstadt (DE); Hey, Joachim, Dr., 90408 Nürnberg (DE)

(57) **Zusammenfassung**

Die vorliegende Erfindung betrifft ein Röntgensystem zum Aufnehmen von Röntgendatensätzen eines Patienten (1), mit einem Röntgengerät (2) zum Aufnehmen von dreidimensionalen Röntgendatensätzen eines Patienten (1) und eine Vorrichtung zum Bereitstellen von Positionsdaten, wobei die Positionsdaten zur Ermittlung der räumlichen Beziehung zwischen mindestens zwei Röntgendatensätzen, die von unterschiedlichen Körperpartien des Patienten (1) aufgenommen wurden, dienen.

Weiterhin betrifft die vorliegende Erfindung ein in diesem Röntgensystem angewendetes Verfahren zur Bestimmung der räumlichen Beziehung von einander unabhängig gemessenen Röntgendatensätzen.

## Beschreibung

Vorrichtung und Verfahren zur Bestimmung der räumlichen Beziehung von einander unabhängig aufgenommenen Röntgendatensätzen (2D oder 3D)

Die vorliegende Erfindung betrifft ein Röntgensystem zum Aufnehmen von Röntgendatensätzen von Patienten sowie ein Verfahren zur Bestimmung der räumlichen Beziehung von einander unabhängig gemessenen Röntgendatensätzen an einem Patienten.

Bei medizinischen bzw. chirurgischen Eingriffen an einem Patienten werden u.a. mobile C-Bogen-Röntgengeräte eingesetzt, mittels derer Bereiche des Patienten röntgenbildlich erfasst werden können. Durch diese Erfassung können dreidimensionale Röntgenaufnahmen gewonnen und auf einem Bildschirm dargestellt werden. Das rekonstruierte Volumen, das mit solchen dreidimensionalen Röntgendatensätzen dargestellt werden kann, beträgt zur Zeit etwa (12cm)³.

In vielen Fällen müssen jedoch mehrere Bereiche des Patienten erfasst werden, die räumlich auseinander liegen. Da die so gewonnenen Röntgendatensätze für bestimmte chirurgische Eingriffe in eine räumliche Beziehung zueinander gesetzt werden müssen, ist eine exakte Kenntnis der räumlichen Positionen der einzelnen Röntgendatensätze erforderlich.

Ein Beispiel hierfür ist ein operativer Eingriff am Knie eines Patienten beim sog. Total Knee Replacement. Hier werden dreidimensionale Röntgenaufnahmen (Röntgendatensätze) vom Knie, vom Fußgelenk und von der Hüfte benötigt, deren räumliche Beziehung zueinander bekannt sein muss, um die richtige Lage- und Winkelposition des Kniegelenks zu erfassen.

Die DE 199 17 867 A1 offenbart ein Verfahren und eine Vorrichtung für eine Lagezuordnung von Behandlungsgeräten zu Röntgenaufnahmen. Die Ermittlung der Positionsdaten des Behandlungsgebiets erfolgt mit einem kameraunterstützten Navigationssystem und mit Hilfe einer gleichzeitig mit einer Röntgenaufnahme des Behandlungsgebietes erfassten Referenzstruktur. Mehrere Röntgenaufnahmen verschiedener Bereiche des Behandlungsgebietes können zu einem Gesamtbild zusammengesetzt werden. Eine direkt am Patienten angebrachte Referenzierungseinrichtung erlaubt ein Bewegen des Patienten zwischen zwei Röntgenaufnahmen, wenn diese Referenzierungseinrichtung vom Navigationssystem verfolgt werden kann.

Die Aufgabe der vorliegenden Erfindung ist, ein Röntgensystem und ein in diesem Röntgensystem anwendbares Verfahren zur Verfügung zu stellen, die auf einfache Weise und mit geringem apparativen Aufwand die genaue Bestimmung der räumlichen Beziehung zwischen mindestens zwei Röntgendatensätzen, die von unterschiedlichen Körperpartien eines Patienten aufgenommen wurden, ermöglichen.

Diese Aufgabe wird durch Röntgensysteme gemäß den unabhängigen Ansprüchen 1 und 4 und Verfahren gemäß den unabhängigen Ansprüchen 6 und 7 gelöst. Vorteilhafte Ausgestaltungen der vorliegenden Erfindung sind in den Unteransprüchen angegeben.

Bei der in den unabhängigen Ansprüchen 1 und 6 angegebenen Ausgestaltungen der vorliegenden Erfindung wird der Maßstab in dem Bereich, in dem die Röntgendatensätze aufgenommen werden, angebracht und ist in den aufgenommenen Röntgendatensätzen sichtbar. Der Maßstab ist dabei in Relation zu den Patienten fest angebracht und in allen aufgenommenen Röntgendatensätzen sichtbar.

Der Maßstab kann dabei beispielsweise an dem Patienten selbst angebracht werden oder in dem Operationstisch, auf dem der Patient während des Eingriffes liegt, integriert sein. In jedem Fall muss der Maßstab so angebracht sein, dass er in Relation zum Patienten fest ist.

In der zweiten Ausgestaltung der vorliegenden Erfindung, die in den unabhängigen Ansprüchen 4 und 7 angegeben ist, wird die erfasste räumliche Beziehung der aufgenommenen Röntgendatensätzen zueinander durch eine Verarbeitungsvorrichtung automatisch berechnet und/oder verarbeitet.

Die automatische Verarbeitung der räumlich erfassten Beziehung durch die Verarbeitungsvorrichtung hat den Vorteil, dass diese Beziehungen nicht nur für den Chirurgen als Informationen dargestellt werden kann, sondern als Grundlage für weitere Berechnungen dienen kann. So kann auf diese Weise eine Vergrößerung des Volumens der aufgenommenen dreidimensionalen Röntgendatensätzen erreicht werden. Dazu werden mehrere dreidimensionale Röntgendatensätze aufgenommen, die räumlich aneinandergrenzen oder sich räumlich ein wenig überlappen, durch die Verarbeitungsvorrichtung zusammengefügt und auf dem Bildschirm dargestellt.

Die vorliegende Erfindung wird nachfolgend anhand bevorzugter Ausführungsbeispiele unter Bezug auf die beigefügten Zeichnungen näher erläutert, in denen zeigen:
- Fig. 1: eine Darstellung des erfindungsgemäßen Röntgensystems gemäß einer ersten Ausgestaltung der vorliegenden Erfindung, und
- Fig. 2: eine Darstellung des erfindungsgemäßen Röntgensystems gemäß einer zweiten Ausgestaltung der vorliegenden Erfindung.

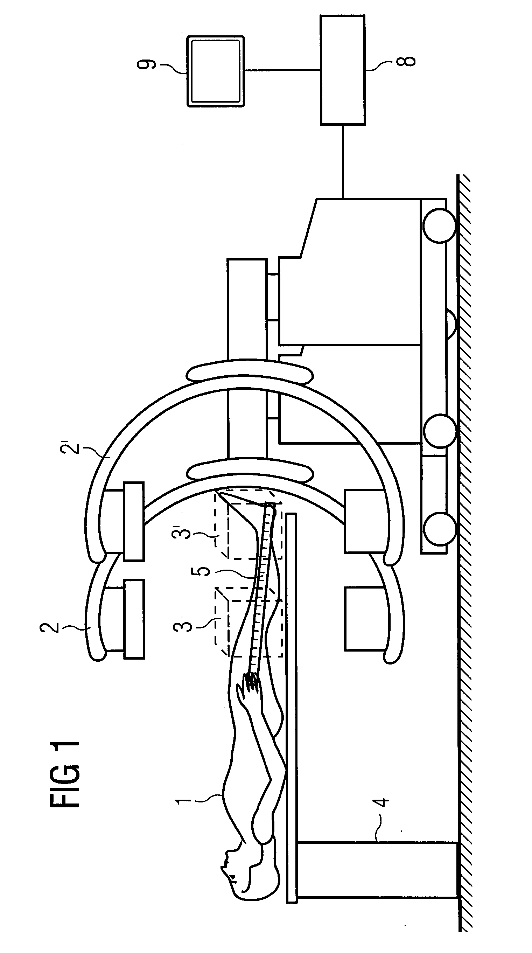

Anhand von Fig. 1 wird nachfolgend die Funktionsweise eines C-Bogen-Röntgengerätes 2 (nachfolgend Röntgengerät 2 genannt) und eine erste Ausgestaltung der vorliegenden Erfindung detailliert beschrieben.

Die dreidimensionalen Röntgendatensätze werden gewonnen, indem zuerst durch das Röntgengerät 2 eine Vielzahl von zweidimensionalen Röntgenbildern von einem bestimmten Bereich des Patienten 1, der auf einem Operationstisch 4 liegt, aufgenommen werden. Dabei werden durch den C-Bogen des Röntgengeräts 2 von dem bestimmten Bereich (z.B. Knie) eine Vielzahl von zweidimensionalen Röntgenbildern unter verschiedenen Winkeln aufgenommen. Die so gewonnenen zweidimensionalen Röntgendatensätze werden durch einen Computer unter Anwendung bekannter Projektionsmatrizen in mindestens einen dreidimensionalen Röntgendatensatz projiziert. Der dreidimensionale Bereich (Volumen), der auf diese Weise aufgenommen und dargestellt werden kann, wird z. B. als Würfel mit dem Bezugszeichen 3 dargestellt.

Der dreidimensionale Röntgendatensatz wird nun auf einer Anzeigevorrichtung 9 (Bildschirm) angezeigt, wobei sich ein Benutzer (i.d.R. der Chirurg, der den operativen Eingriff an dem Patienten 1 durchführt) sich den aufgenommenen Bereich des Patienten beispielsweise aus verschiedenen Blickwinkeln und/oder in verschiedenen Ebenen ansehen kann.

Gemäß dieser Ausgestaltung der vorliegenden Erfindung wird ein in Röntgenbildern (Röntgendatensätzen) sichtbarer Maßstab 5 in den Bereich eingebracht, der mittels der Röntgenbildern sichtbar gemacht werden soll. Dabei muss der Maßstab nicht unbedingt in dem projizierten dreidimensionalen Röntgendatensätzen für den Chirurgen sichtbar sein. Es genügt auch, wenn der Maßstab 5 bei der Aufnahme der zweidimensionalen Röntgendatensätze in einem oder wenigen Röntgendatensätzen enthalten ist.

Der Maßstab 5 wird in diesem Fall nicht im projizierten dreidimensionalen Röntgendatensatz angezeigt, jedoch kann auf diese Weise der Maßstab 5 von der Verarbeitungsvorrichtung 8 automatisch erkannt und verarbeitet werden. Sichtbar im Sinne der vorliegenden Erfindung bedeutet also, dass der Maßstab für das Röntgengerät 2 "sichtbar" ist, d.h. dass der Maßstab in mindestens einem zweidimensionalen Röntgendatensatz enthalten ist, und somit die daraus gewonnene Information weiterverarbeitet werden kann.

Unter einem Maßstab 5 wird im Sinne der vorliegenden Erfindung Markierungen verstanden, die eine genau definierte räumliche Beziehung zueinander haben. Das können einzelne Markierungen sein, die in bestimmten, festgelegten Abständen am Patienten 1 oder am Operationstisch 4 angebracht werden. Das kann aber auch, wie in Fig. 1 dargestellt, eine Art Meßlatte mit entsprechenden, in den Röntgendatensätzen sichtbaren, Markierungen sein. Der Maßstab 5 kann dabei beispielsweise in einer Linie oder in einem zweidimensionalen Raster abgebildet sein.

Werden jetzt an unterschiedlichen Positionen des Körpers des Patienten 1 Röntgendatensätze aufgenommen, so muss der durchgängige Maßstab in allen Positionen (für den Chirurgen und/oder für die Verarbeitungsvorrichtung 8) erkennbar sein. Durch die räumliche Lage des Maßstabs 5 in den einzelnen 3D-Volumina 3, 3' in Verbindung mit einer erkennbaren Einteilung, beispielsweise in Zentimeter (cm), auf dem Maßstab sind die räumlichen Beziehungen der einzelnen dreidimensionalen Aufnahmen (Röntgendatensätze) einfach bestimmbar. Die verschobene Position des Röntgengeräts 2 und somit des aufgenommenen (bzw. aus den zweidimensionalen Röntgendatensätzen rekonstruierten) Volumens 3 sind in der Figur mit 2' und 3' gekennzeichnet.

Vorteilhafterweise ist der Maßstab 5 weitgehend durchlässig für Röntgenstrahlen, damit das Ergebnis der aufgenommenen Röntgendatensätze und somit der daraus resultierende dreidimensionale Röntgendatensatz möglichst wenig verfälscht wird.

Die Vorrichtung, die die Berechnung der zweidimensionalen Röntgendatensätze in einen (oder auch mehrere) dreidimensionalen Röntgendatensatz durchführt, und die Verarbeitungsvorrichtung 8 gemäß der vorliegenden Erfindung sind vorteilhafter Weise in einem Gerät (Computer) kombiniert.

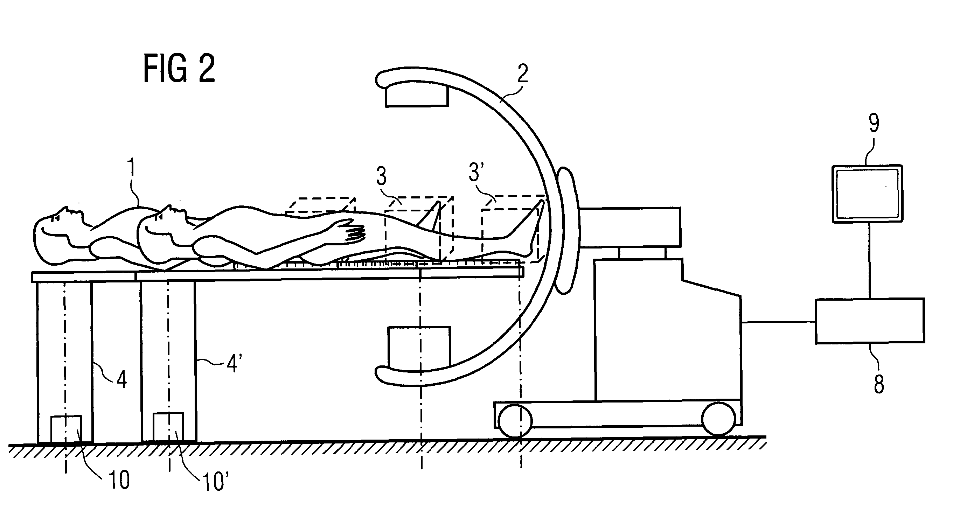

Fig. 2 zeigt eine Darstellung des erfindungsgemäßen Röntgensystems gemäß einer zweiten Ausgestaltung der vorliegenden Erfindung.

Die Bezugszeichen in Fig. 2 sind mit den Bezugszeichen der Fig. 1 identisch. In dieser Ausgestaltung der vorliegenden Erfindung besteht jedoch die erfindungsgemäße Vorrichtung zum Bereitstellen von Positionsdaten aus einer in einem verfahrbaren/verschiebbaren Operationstisch 4 integrierten Einrichtung zum Erfassen der Bewegung des Operationstisches 10, die eine Bewegung des Tisches quantitativ registriert; die verschobene Position des Operationstisches 4 wird mit dem Bezugszeichen 4' symbolisiert.

Das Röntgengerät 2 ist in diesem Ausführungsbeispiel fest installiert.

Die Relativbewegung vom C-Bogen zum Patienten kann wiederum durch einen beispielsweise im Operationstisch 4 integrierten Maßstab erfolgen und/oder durch Messung der Tischbewegung. Nachdem die Aufnahme des dreidimensionalen Röntgendatensatzes an einer ersten Position erfolgt ist, z.B. Knie, erfolgt ist fährt der Tisch den Patienten an eine zweite Position, so dass zum Beispiel die Hüfte im Isozentrum des C-Bogens liegt; der C-Bogen steht dabei fest. Dann stoppt die Tischbewegung, die zurückgelegte Strecke des Operationstisches 4 wird gemessen und es erfolgt eine weitere Aufnahme eines dreidimensionalen Röntgendatensatzes (im Beispiel von der Hüfte).

## Patentansprüche

1. Röntgensystem zum Aufnehmen von Röntgendatensätzen eines Patienten (1), mit einem Röntgengerät (2) zum Aufnehmen von Röntgendatensätzen eines Patienten (1),
**gekennzeichnet durch**
einen in Relation zum Patienten festen Maßstab (5), der zumindest in einem Teil der Röntgendatensätze sichtbar ist, und zur Ermittlung der räumlichen Beziehung zwischen mindestens zwei Röntgendatensätzen, die von unterschiedlichen Körperpartien des Patienten aufgenommen wurden, dient.

2. Röntgensystem gemäß Anspruch 1,
**dadurch gekennzeichnet,**
**dass** der Maßstab (5) fest an dem Patienten (1) angebracht ist.

3. Röntgensystem gemäß Anspruch 1 oder 2,
**dadurch gekennzeichnet,**
**dass** der Maßstab (5) an einem Operationstisch (4) für den Patienten angebracht ist, wobei der Maßstab in Relation zu dem Patienten (1) fest angebracht ist.

4. Röntgensystem zum Aufnehmen von Röntgendatensätzen eines Patienten (1), mit
einem Röntgengerät (2) zum Aufnehmen von Röntgendatensätzen eines Patienten (1) und einem Operationstisch (4, 4') zur Positionierung des Patienten für das Aufnehmen von Röntgendatensätzen,
**gekennzeichnet durch**
eine Einrichtung (10) zum Erfassen der Bewegung des Operationstisches (4, 4'), wobei die Einrichtung Positionsdaten zur Ermittlung der räumlichen Beziehung zwischen mindestens zwei Röntgendatensätzen, die von unterschiedlichen Körperpartien des Patienten aufgenommen wurden, **durch** Registrieren einer Bewegung des Operationstisches (4, 4') zwischen den Aufnahmen quantitativ in Relation zu dem Röntgengerät (2), bereitstellt.

5. Röntgensystem gemäß einem der Ansprüche 1 bis 4, **gekennzeichnet durch**
eine Verarbeitungsvorrichtung (8) zum automatischen Verarbeiten der bereitgestellten Positionsdaten.

6. Verfahren zur Bestimmung der räumlichen Beziehung von einander unabhängig gemessenen Röntgendatensätzen, wobei Röntgendatensätze eines Patienten (1) aufgenommen werden,
**dadurch gekennzeichnet,**
**dass** die Ermittlung der räumlichen Beziehung zwischen mindestens zwei Röntgendatensätzen, die von unterschiedlichen Körperpartien des Patienten aufgenommen wurden, mittels eines in Relation zum Patienten festen Maßstabs (5) erfolgt, der in den aufgenommenen Röntgendatensätzen sichtbar ist.

7. Verfahren zur Bestimmung der räumlichen Beziehung von einander unabhängig gemessenen Röntgendatensätzen, wobei Röntgendatensätze eines Patienten (1) aufgenommen werden,
**dadurch gekennzeichnet,**
**dass** die Ermittlung der räumlichen Beziehung zwischen mindestens zwei Röntgendatensätzen, die von unterschiedlichen Körperpartien des Patienten aufgenommen wurden, mittels der Registrierung einer Bewegung des Operationstisches (4, 4') zwischen den Aufnahmen quantitativ in Relation zu dem Röntgengerät (2) erfolgt.

8. Verfahren gemäß einem der Ansprüche 6 oder 7, **gekennzeichnet durch**
eine automatische Weiterverarbeitung der bereitgestellten Positionsdaten.
